# EUROPEAN PATENT APPLICATION

(11) **EP 1 176 197 A1**
(43) Date of publication of application: **30.01.2002**
(21) Application number: 00202084.0
(22) Date of filing: 16.06.2000
(51) Int. Cl.: C12N 15/12, A01K 67/027, C12N 5/06, A61K 31/00, C07K 14/705

(54) **A transgenic animal model for cardiac arrhythmias**

(71) Applicant: Vlaams Interuniversitair Instituut voor Biotechnologie vzw., 9052 Zwijnaarde (BE); Leuven Research & Development vzw, 3000 Leuven (BE)
(72) Inventor: The designation of the inventor has not yet been filed
(74) Representative: Brants, Johan Philippe Emile

(57) **Abstract**

This invention relates to a transgenic animal model and cell lines derived thereof useful in the testing of molecules for their efficacy in the treatment of cardiac arrhythmias. The present invention provides a reliable model bearing a specific mutation (nine basepair deletion in the SCN5A gene) in a cardiac sodium channel, responsible for cardiac arrhythmias, and mimics in particular the clinical and electrophysiological characteristics of Long QT syndrome.

## Description

### Field of the invention

This invention relates to a transgenic animal model and cell lines derived thereof useful in the testing of molecules for their efficacy in the treatment of cardiac arrhythmias. The present invention provides a reliable model bearing a specific mutation (nine basepair deletion in the SCN5A gene) in a cardiac sodium channel, responsible for cardiac arrhythmias, and mimics in particular the clinical and electrophysiological characteristics of Long QT syndrome.

### Background of the invention

Ion channels are proteins that reside in the cell membrane. In response to external stimuli, such as changes in electrical potential across the cell membrane, ion channels can form a pore which allows movement of ions (Na⁺ or Ca²⁺ or K⁺ or Cl⁻) in or out of cells. The integrated behaviour of thousands of ion channels in a single cell results in an ion current, and the integrated behaviour of many ion currents makes up the characteristic cardiac action potential. Thus, ion channels are the fundamental building blocks that determine the electrical activity of for example cardiac tissue. Arrhythmias represent the end product of abnormal ion-channel structure, number, or function. These abnormalities can be primary, or may be secondary induced by other factors in the cell's environment, such as acute ischemia, changes in circulating neurohormones, or intracellular calcium overload, which can modify the channel function.
An example of defects in ion channels in the heart is Long QT syndrome (LQT) which is an inherited cardiac disorder affecting at least 1 in every 5,000 live births in Westernized societies. A recently published multi-center study on 35,000 newborns demonstrated that the LQT syndrome is one of the major causes contributing to sudden infant death ("still birth")¹³. In addition, another large fraction (probably ∼50%) of affected patients survives for longer periods, but then suddenly dies due to fatal arrhythmia. Sadly, patients affected by LQT syndrome are often young, otherwise healthy people, explaining the dramatic socio-economic impact of this disorder. Activation of the sympathic nervous system by physical or emotional stress often precipitates the arrhythmia during childhood, whereas in other patients, the arrhythmogenia are triggered at rest or during sleep ("nocturnal sudden death syndrome").¹⁴ Recent evidence suggests that these different phenotypic forms of LQT are related to specific genetic mutations, that is, the stress-induced arrhythmia may occur more frequently in patients with potassium channelopathies, whereas the sleep-associated arrhythmia are more frequent in patients with sodium channelopathies.¹⁵ LQT can also be acquired, usually as a result of treatment with certain medications, including antibiotics, antihistamines, general anesthetics and, most commonly, antiarrhythmic drugs.² Because of the widespread and frequent use of these drugs, LQT represents a significant threat to public health. Clinically, patients with LQT suffer hypotension, syncope, seizures and sudden death as a result of polymorphic ventricular tachyarrhythmia ("torsade de pointes") or fibrillation. Electrocardiographically, LQT is characterized by a prolongation of the QT-interval (a temporal measure of cardiac repolarization), sinus pauses and abnormal T-wave morphology. At present, therapeutic management of patients with a LQT syndrome is problematic. Indeed, β-blockers, pacemakers and ganglionectomy all have limitations, and fail in more then 20% of the patients.^{9, 14} The safest way to prevent development of lethal arrhythmia is the use of an implantable defibrillator, an invasive, costly devise which has limited applicability in the newborn. Because of the specific genotype-phenotype relationship, a treatment specific for each channelopathy appears to be mandated.

Over the past 10 years, the number of LQT patients has increased dramatically. It is doubtfull that this reflects an increase in incidence. Rather, it can be explainded by an emerging awareness of, and respect for, this cardiac channelopathy. Nevertheless, the understanding of the molecular and cellular mechanisms triggering the fatal event of arrhythmia is poor. This is further complicated by the complex genotype-phenotype relationship. Indeed, the incidence of sudden death and QT-prolongation is variable and sometimes subtle, and in addition, many LQT gene carriers have relatively normal QT intervals, exhibiting as a first disease sign, fatal cardiac arrhythmia. Furthermore, it can be speculated that inheritable malfunctioning of ion channels may constitute a possible etiological basis for the unexplained 300,000 sudden deaths due to ventricular fibrillation each year in the USA alone. In fact, the LQT syndrome is the most common cause of sudden death in the population under the age of 20 years.

Historically, the LQT syndrome has been classified in two groups according to its inheritance pattern: (i) an autosomal dominant pattern of inheritance, also called the Romano-Ward syndrome, and (ii) a less frequent autosomal recessive variant of the LQT syndrome, termed the Jervell and Lange-Nielsen syndrome, with a high incidence of sudden death and congenital neural deafness.

Since LQT is associated with abnormal cardiac repolarization, genes encoding ion channels or modulating ion channel function, have been considered as putative candidates. Recent genetic analyses have identified at least five chromosomal loci with more than 40 mutations that can cause LQT. This genetic heterogeneity may explain the variable expression of the phenotype ¹.

The affected genes, thus far known to be implicated in LQT development, include:
- KVLQT1, a voltage-activated potassium channel subunit. The gene is located on chromosome 11p15.5, and its defects are known to cause LQT type-1 (or LOT1)¹⁶.
- IsK (also called KCNE1), a regulatory subunit (termed as minK), that together with KVLQT1, constitutes the channel for the slow component of the delayed rectifier current I_{Ks}. The gene is located on chromosome 21, and its defects are known to cause LQT5⁷.
- HERG, a potassium channel on chromosome 7q35-36, responsible for the rapid component of the delayed rectifier K⁺ current I_{Kr}.
   Its defects are known to cause LQT2 ¹⁷.
- SCN5A, the cardiac voltage-gated sodium channel, the gene of which is located on chromosome 3p21-24. Its defects are known to cause LQT3¹⁸.
- An unknown gene on chromosome 4q25-27, wich most likely encodes a Ca²⁺/calmodulin-dependent protein kinase II. Its defects are known to cause LQT4 ¹⁹.

There exists significant variability in the penetrance and severity of the clinical disease, caused by these specific gene mutations (genotype-phenotype relationship). For example, mutations in SCN5A or HERG are associated with the dominant Romano-Ward variant of LQT, whereas KVLQT1 and IsK have been implicated in both the Romano-Ward and in the recessive Jervel and Lang-Nielsen variant of LQT²⁰.

Increasing evidence is being provided that the sodium channelopathy of LQT3 is a severe and fatal variant of the LQT syndrome, although its precise incidence remains to be determined.²¹ Voltage-gated sodium (Na⁺) channels are membrane-spanning proteins mediating rapid depolarization and spreading of excitation in ventricular and atrial myocytes and in Purkinje fibers. Na⁺ channels can conduct a small inward "window" or "slowly inactivating" current during the plateau phase of the action potential that can prolong the duration of the action potential. The voltage-gated Na⁺ channel is a heterotrimeric protein consisting of a large α-subunit (227 kDa) and two smaller β-units (β₁ 36-kD and β₂ 33-kD). Sodium channel α-subunits consist of four homologous domains (DI-DIV) that each contain 6 transmembrane segments joined by short intracellular domains (ID1-2, ID2-3, ID3-4). The human *SCN5A* gene is a member of a multigene family of voltage-gated Na⁺ channel α-subunits, of which at least nine distinct isoforms have been identified. The *SCN5A* gene comprises 28 exons, spanning approximately 80 kb on chromosome 3p21 and encoding a protein of 2016 amino acids.²²

LQT3 is caused by *SCN5A* gene alterations that involve a 9-basepair deletion (encoding amino acids KPQ at positions 1505-1507)¹⁰ or four other point mutations (R1623Q, R1644H, N1325S, D1790G), which all affect the transmembrane domains III and IV in a region critically important for the fast inactivation of the Na⁺ current.¹⁰ When expressed in *Xenopus* oocytes, each of these gene alterations generates inactivation-resistant whole-cell current, suggesting that the mechanism of LQT3 is persistence of a depolarizing sodium current during the plateau phase of the action potential (slow inactivating current). This persistence is due to the occurrence of brief (0.3 ms) dispersed openings and long-lasting (5 ms) bursts of openings of individual sodium channels. Similar mutations are also found in a homologous voltage-gated sodium channel in skeletal muscle which are responsible for hyperkalemic periodic paralysis. Identification of the specific channelopathy in affected patients would allow a better and more specific drug treatment. The recent molecular break-throughs in identifying the etiological basis of LQT have provided new opportunities to investigate the genotype-phenotype correlations in more detail 23. US patent 5,599,673 describes the identification of the molecular basis of LQT syndrome and its use in diagnosis.

Transgenic mice are suitable models to test the phenotypic consequences of specific genetic alterations. Although transgenic mice have been generated with engineered dysfunctions in potassium channels (encoded by KVLQT1, HERG and ISK), they do not resemble the entire clinical spectrum of the clinically observed LQT syndrome. For example, Barry et al.⁶ generated transgenic mice overexpressing a dominant negative Kv4 α transgene, leading to prolongation of the QT-interval, but without ventricular arrythmias, whereas Babij et al.⁵ overexpressed a dominant negative HERG G628S mutation leading to prolongation of the action potential duration at the single cell level, but without prolongation of the QT-interval at the organ level or arrhythmia. Drici et al. 7, ²⁴ described the generation of a murine model of the Jervell and Lange-Nielsen variant of the LQT syndrome by targeted disruption of the IsK gene. These mice exhibited an exacerbated adaptation of the QT-interval to the heart rate, explaining the greater susceptibility to ventricular arrhythmia as seen in patients lacking Iₖₛ. Finally, London et al.⁸ generated transgenic mice overexpressing a dominant negative Kv1.1 α transgene, thereby reducing the I_{TO} and causing prolonged ventricular action potentials and ventricular tachyarrhytmia.

Presently there is no experimental mouse model available for the sodium channel dysfunction variant of LQT. Therefore, it is a specific object of the current invention to provide a transgenic mouse model expressing a modified SCN5A sodium channel gene (KPQ-deletion) similar to that observed in patients. Experiments carried out in the prior art indicate that transgenic mice cannot be a reliable model for cardiac arrhythmias because of the differences between the cardiac physiology between human and mouse hearts^{5,6,7,8,24}. However, unexpectedly the current animal model reflects the entire clinical and electrophysiological spectrum of symptoms as found in patients. Therefore, said model of LQT will be extremely useful not only to improve our understanding of the molecular and cellular mechanisms contributing to cardiac arrhytmias, but in addition to design and to test therapies for this life threatening condition.

### Aims of the invention

The present invention aims at providing research tools, animal and cellular models and therapeutics for patients suffering from cardiac arrhytmias. The invention particularly aims at providing a non-human transgenic animal for cardiac arrhytmia and specifically a mouse transgenic animal for long QT syndrome. The present invention further aims at providing cells derived from said transgenic animal such as stem cells, embryonic stem cells and cardiomyocytes. The invention further aims at providing screening assays for the identification of therapeutic molecules that can be used for the preparation of a medicament for the treatment of cardiac arrhythmias. Finally the invention aims at using the transgenic animal model for the monitoring of cardiac arrhythmogenic side effects of medicaments.

### Figure Legends

### Figure 1:

ΔKPQ.SCN5A targeting vector contains exon 24, 25, 26 and 27. Exon 26 harbours the KPQ deletion. After homologous recombination, the Neomycin resistance cassette can be removed through Cre mediated excision.

### Figure 2:

Illustration of a Signal Averaged ElectroCardioGram (SAECG) complex of lead II. As one can observe the biphasic T wave is longer and taller in LQT3. The arrow indicates the QTmax, the vertical line, after QTmax, where the first derivative becomes zero, indicates the end of the T-wave.

### Figure 3:

This figure summarizes SAECG measurements: Values are averaged over all 8 leads. All values between WT and LQT3 are statistically different from each other (P<0.05), except PR-interval values.

### Figure 4:

This strip represents a 4 sec. 8 lead (I, II, III, 4VR, aVL, aVF, V2, V6) recording, as was observed in an anaesthetized LQT3 mouse under baseline conditions. The QRS axis is constantly changing direction. This polymorph tachycardia resembles the life threatening Torsade de Pointes as is seen in LQTS patients.

### Detailed description of the invention

The present invention provides a transgenic animal model, and cells derived thereof, for cardiac arrhythmias and more specifically for Long QT syndrome (LQTS). LQTS is an inherited cardiac arrhythmia that causes abrupt loss of consciousness, syncope, seizures and sudden death from ventricular tachyarrhythmias. Most LQTS gene carriers manifest prolongation of the QT interval on electrocardiograms, a sign of abnormal cardiac repolarization. The clinical features of LQTS result from episodic cardiac arrhythmias, specifically torsade de pointes, named for the characteristic undulating nature of the electrocardiogram in this arrhythmia. Torsade de pointes may degenerate into ventricular fibrillation, a particularly lethal arrhythmia. Although LQT is not a common diagnosis, ventricular arrhythmias are very common, more than 300.000 US citizens die suddenly every year and, in many cases, the underlying mechanism may be aberrant cardiac repolarisation. The present animal model therefore provides a unique opportunity to study life-threatening cardiac arrhythmias at the molecular level.

In a first embodiment a non-human transgenic animal is provided with a deletion of the essential KPQ amino acids in the gene product encoded by the SCN5A gene. The mutation in this document is designated as SCN5A^{m}, SCN5A^{m/+} is a heterozygous transgenic animal or cell line and SCN5A^{m/m} is a homozygous transgenic animal or cell line. The 'specific deletion' involves a 9-basepair deletion, encoding amino acids KPQ. In mouse, human and rat the amino acids KPQ are at positions 1505-1507 corresponding with the numbering of the amino acids of the translated cDNA of the *SCN5A* gene. Said 9-basepair deletion in the human SCN5A gene causes in man the LQT3 syndrome which is the most severe clinical phenotype of LQT syndrome. The animal model is preferably from a genus selected from the group consisting of *Mus* (e.g. mice), *Rattus* (e.g. rats), *Oryctologus* (e.g. rabbits) and *Mesocricetus* (e.g. hamsters). More preferably the animal is a mouse. The specific deletion was performed in mouse embryonic stem cells by Cre/Lox mediated homologous recombination. The mice, homozygous for said mutation, die early during embryonic development (Embryonic day 10). Consequently only mice heterozygous for said mutation could be generated. In contrast with current models of Long QT syndrome the present model has some major advantages: (1) electrocardiographic (ECG) measurements show abnormalities as observed in LQT3 patients and (2) severe spontaneous arrhytmias are found. This novel transgenic mouse model is useful to assay for molecules and test experimental therapies. Furthermore, transgenic mice offer the additional advantage for large scale-testing of anti-arrhythmic drugs because of their small size and fast breeding propensities. Said mouse model is also likely to yield novel insights in the molecular mechanisms of how aberrant cardiac repolarisation occurs and how the sodium channel functions. Indeed, electrophysiological characterization of the affected sodium channel in human heart has not been studied, likely because of the practical and ethical problems of using human cardiac tissue samples. In addition, this transgenic mouse model will allow the study of the pathophysiology of this channel in a more physiological environment within cardiomyocetes *in vivo,* as compared to the over-expression of the modified sodium channel in heterologous cultured cells *in vitro.*

In a specific example it may be possible to excise the mutated SCNSA^{m} gene for use in the creation of transgenic animals containing the mutated gene. In a further example, an entire human SCN5A^{m} allele may be cloned and isolated, either in parts or as a whole, in a cloning vector (e.g. cosmid or yeast or human artificial chromosome). The human variant SCN5A^{m} gene, either in parts or in whole, may be transferred to a host non-human animal, such as a mouse or a rat. As a result of the transfer, the resultant transgenic non-human animal will preferably express one or more mutant SCN5A^{m} polypeptides. Most preferably, a transgenic non-human animal of the invention will express one or more mutant SCN5A^{m} polypeptides in a heart-specific manner. Alternatively, one may design minigenes encoding variant SCN5A^{m} polypeptides. Such mini-genes may contain a cDNA sequence encoding a variant SCN5A^{m} polypeptide, preferably full-length, a combination of SCN5A^{m} gene exons, or a combination thereof, linked to a downstream polyadenylation signal sequence and an upstream promoter (and preferably enhancer), preferably such a promoter is cardiac specific. Such a mini-gene construct will, when introduced into an appropriate transgenic host (e.g., mouse or rat), express an encoded variant SCN5A^{m} polypeptide, preferably in the heart.

In a further embodiment cell lines can be derived from the transgenic animal model. Derived cell lines can be heterozygous or homozygous for the above described mutation in the SCN5A gene. Typical but not limited examples of cell lines are fibroblasts and keratinocytes but preferably cell lines are cardiomyocytes. For a correct ion channel function it is preferred that the cells are derived from the heart, e.g. cardiomyocytes or progenitor cells such as cardiomyoblasts, in order to provide the precise intracellular milieu and regulatory factors that determine ultimate ion channel function. Cardiomyocytes derived from SCN5A^{m/+} mice can be efficiently derived using collagenase-based cell isolation procedures known in the art. Since SCN5A^{m/m} mice are lethal at Embryonic day 10, is it not possible to obtain homozygous mutant cardiomyocytes from transgenic animals. Therefore it is possible to start from homozygous mutant embryonic stem (ES) cells which can be efficiently differentiated to cardiomyocytes *in vitro.* Said ES cells can be isolated from homozygous mutant transgenic embryos. It is well known in the art that cells from the inner cell mass of mammalian blastocysts can be maintained in tissue culture under conditions where they can be propagated indefinitely as pluripotent embryonic stem (ES) cells (Thomson *et al*., 1998, Science 282, 1145). As such blastocysts from double homozygous mutant mice can be rescued from 2 days old embryos. Alternatively, homozygous mutant ES cells can be generated by high G148 selection of the heterozygous mutant ES cells. SCN5A^{m/m} embryonic stem cells can then be induced to differentiate into pure cultures of cardiomyocytes using the following genetic selection procedure⁵⁹. SCN5A^{m/m} embryonic stem cells are transfected with a puromycin resistance expression cassette driven by the cardiac α-myosin heavy chain promoter. When the latter becomes active during cardiogenesis *in vitro,* puromycin selection will be initiated, resulting in the selective survival of pure cardiomyocytes. An enhanced green fluorescent protein gene cloned as a bicistronic message after the puromycin gene is used to visualize the differentiated cardiomycytes. A hygromycin resistance expression cassette is used for stable co-transfection of the stem cells.
In another embodiment several types of stem cells can be isolated from the transgenic animal such as angioblasts or cardiomyoblasts, respectively progenitor stem cells for endothelial cells and cardiomyocytes.

In a specific embodiment SCN5A^{m/m}cardiomyocytes can be used to study the impairment of the inactivation of sodium ion channels which also occurs during ischemia. The precise mechanism is not understood but presumably lipid metabolites, which are liberated during hypoxia, can impair sodium channel inactivation. Both the genetic alteration and hypoxia result in sodium overload of the cardiomyocyte. Therefore, the SCN5A^{m/m} cardiomyocytes may also provide a suitable model to study the consequences of the impaired sodium channel inactivation on myocyte function, as also found during ischemia. The relevance of hypoxia-induced alterations in sodium channel function is illustrated by the high incidence of arrhythmias found in patients suffering from myocardial ischemia.

In another embodiment cell lines described above can be used for the development of a method for the screening of molecules and monitoring the effectiveness of said molecules. It is expected that homozygous mutant cell lines are a better model than heterozygous mutant cell lines because the latter cell lines have a mixed genetic background of SCN5A gene expression: wild type SCN5A and SCN5A^{m}. Alternatively cell lines transfected with the human SCN5A^{m} gene offer an alternative model for screening. Since the SCN5A^{m} mutation is dominant, suitable cell lines can also be transfected with this mutant gene and used for screening purposes. Other cell systems, often used in electrophysiological studies, are oocytes of *Xenopus laevis.* In oocytes the mutated SCN5A gene can for example be introduced by microinjection.

In a specific embodiment the cells can also be naturally derived. For example blood samples or other cells derived from the transgenic animal model can be obtained in order to provide the necessary cells which can be permanently transformed into for example a lymphoblastoid cell line using, for example, Epstein-Barr virus. Once established, such cell lines can be grown continuously in suspension culture and may be used for a variety of *in vitro* experiments to study SCN5A^{m} expression and/or the development of screening methods.
Monitoring can be done, following administration of molecules, by electrofysiological methods known in the art for example by patch clamp analysis. With said analysis the electrophysiological characteristics of the modified Na⁺-channel in SCN5^{m/m} cells can be studied, preferably said cells are cardiomyocytes. The Na⁺-current can be measured using the whole-cell and the cell-attached modes of the patch clamp technique. This allows to determine possible genotypic changes in steady-state voltage-dependence of activation-inactivation, in the time course of (in)activation and recovery from inactivation, and in the responsiveness to molecules and candidate antiarrhythmic agents. At the single channel level, opening and closing kinetics of the channel can be determined.
Alternatively Ca²⁺-transients can be monitored using fluorescent dyes such as Fura-2, Indo-1 and derivatives thereof. Fura-2 and indo-1 are UV light-excitable, ratiometric Ca²⁺ indicators that are generally considered to be interchangeable in most experiments. Said fluorescent dyes can be purchased from Molecular Probes Inc. (http://www.probes.com/handbook/sections/2002.html). It is observed that Na⁺ overload in cardiomyocytes with LQT-mutations, leads to Ca²⁺ - overload and said fluctuations of Ca²⁺ can be easily measured with said fluorescent dyes. Alternatively changes in membrane potential of the cells can be measured using fluorescent voltage-sensitive dyes. To maximize the fluorescent signal "slow" dyes can be used, i.e. dyes that respond on the time scale of seconds. These dyes generate a larger change in fluorescence for a similar depolarisation compared to the "fast" dyes. Examples, but not limited to these, of "slow" dyes are oxonols such as B-413, B-436 and B-438 which can be obtained from Molecular Probes Inc. Still another possibility for monitoring known in the art is the use of tracer methods. As an example the Rubidium uptake is measured. The latter methods of monitoring can be easily adapted to a high throughput system.
Suitable molecules for screening may be small molecules, biological polymers, such as polypeptides, polysaccharides, polynucleotides, and the like. Small molecules, e.g. small organic molecules, and other drug candidates can be obtained, for example, from combinatorial and natural product libraries. The test compounds will typically be administered to the culture-medium at a concentration in the range from about 1nM to 1mM, usually form about 10 µM to 1mM. Monitoring can be measured using standard biochemical and electrophysiological techniques. These assays may be performed using conventional techniques developed for these purposes in the course of screening. To perform drug screening assays, it is feasible to accommodate automation of the assay. Interaction (e.g., binding of) between the recombinant cells and the target molecules can be accomplished in any vessel suitable for containing the reactants. Examples of such vessels include microtiter plates, test tubes, and microcentrifuge tubes.

In another embodiment a screening method can be developed with the above described transgenic animal model for the screening of therapeutic molecules for use in the treatment of Long QT syndrome and/or cardiac arrhythmias. Molecules as described above or successful molecules isolated from the cellular screening assays or existing (pre)clinical medicaments can be administrated to the transgenic animal models described before. Monitoring can be done for example by electrocardiographic measurements as examplified further.

In another embodiment the present transgenic animal model can be used for the evaluation of possible cardiac arrhytmogenic side effects of new or existing medicaments. Indeed the present mouse model is more prone to cardiac arrhytomogenicity than the wild type mouse and hence said model is useful for the early evaluation and detection of possible negative arrhythmogenic side effects of medicaments and/or combinations thereof. Usually said side effects or not easily detected in a clinical study before a new medicine enters the market. It is known that several existing medicaments (such as antidepressants, antihistaminica and antibiotics) can prolong the QT-interval and/or induce Torsade de Pointes. As an example it was recently found that the gastrointestinal medicine Propulsid® (Cisapride) blocks a cardiac potassium channel and consequently can provoke cardiac arrhythmias in certain patients *(Circulation* (1998), 97 (2): 204-210).

In another embodiment the molecules obtained in the above described screening methods can be used for the preparation of a medicament. The term 'medicament for treatment' relates to a composition comprising molecules as described above and a pharmaceutically acceptable carrier or excipient (both terms can be used interchangeably) to treat cardiac arrhytmias and long QT syndrome as described fully above. Suitable carriers or excipients known to the skilled man are saline, Ringer's solution, dextrose solution, Hank's solution, fixed oils, ethyl oleate, 5% dextrose in saline, substances that enhance isotonicity and chemical stability, buffers and preservatives. Other suitable carriers include any carrier that does not itself induce the production of antibodies harmful to the individual receiving the composition such as proteins, polysaccharides, polylactic acids, polyglycolic acids, polymeric amino acids and amino acid copolymers. The 'medicament' may be administered by any suitable method within the knowledge of the skilled man. The preferred route of administration is parenterally. In parental administration, the medicament of this invention will be formulated in a unit dosage injectable form such as a solution, suspension or emulsion, in association with the pharmaceutically acceptable excipients as defined above. However, the dosage and mode of administration will depend on the individual. Generally, the medicament is administered so that the molecule obtained by the above described screening method is given at a dose between 1 µg/kg and 10 mg/kg, more preferably between 10 µg/kg and 5 mg/kg. Preferably, it is given as a bolus dose. Continuous infusion may also be used and includes continuous subcutaneous delivery via an osmotic minipump. If so, the medicament may be infused at a dose between 5 and 20 µg/kg/minute, more preferably between 7 and 15 µg/kg/minute.

### Examples

### Generation of the LQT3 mouse model

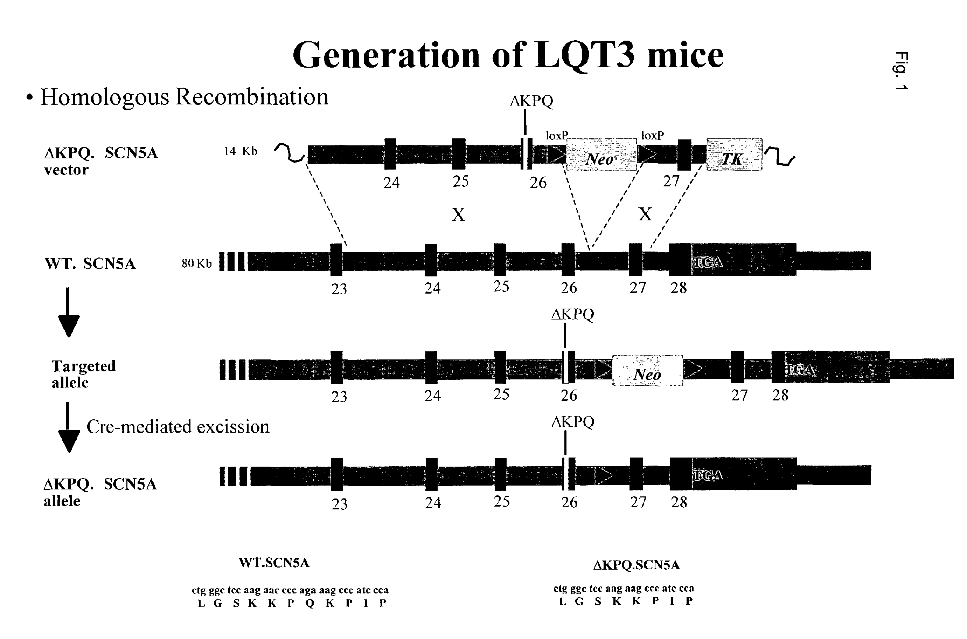

LQT3 is caused by several SCN5A gene alterations. among which a 9-basepair deletion, encoding KPQ-residues. We have generated a LQT3 mouse model that is based on a KPQ deletion in the mouse SCN5A gene (encoding a cardiac specific voltage-gated sodium channel subunit), thereby resembling the sodium channel dysfunction of LQTS in patients. The murine SCN5A gene was isolated to generate a modified SCN5A allele that is lacking the essential KPQ (Lysine, Proline, Glutamine) residues. In order to mutate the SCN5A gene in embryonic stem (ES) cells, a genomic P1 vector containing the murine SCN5A gene (129/SvJ isogenic genetic background; Genome Systems Inc., St. Louis, Missouri) was isolated and mapped by restriction analysis, cloned, and fragments identified for the construction of a SCN5A ΔKPQ targeting vector. This targeting vector (Fig. 1) comprises (from 5' to 3'):
- a 4.7 kb BamHI-Xhol5' homology region comprising exons 24, 25 and 26. Exon 26 lacks the specific nine nucleotide deletion AGAAACCCC (KPQ-deletion), but contains an additional silent mutation to generate an EcoRV site that is used to discriminate the modified from the wild type allele. This was achieved by mutating GACATC to GATATC, at 59 nucleotides upstream from the KPQ deletion.
- a neomycin phosphotransferase (neo) cassette inserted in intron 26 (386 nucleotides downstream of the KPQ-deletion in exon 26). This cassette is flanked by loxP sites to allow subsequent removal via Cre-recombinase.
- a 2.5 kb Kpnl-Sacl 3' homology flank, containing part of intron 26, exon 27 and part of exon 27
- a thymidine kinase gene used for counter-selection.

After electroporation of the pBS:SCN5AΔKPQ targeting vector, homologously recombined ES cell clones, containing both the 9 basepair deletion and the floxed neo-cassette (SCN5A△KPQ-NEO^{m/+}ES cells) were identified by Southern blot analysis and sequencing. A 0.8 Kb Sacl-Xhol fragment, digested from a genomic P1 subclone, containing part of intron 27, was used as external probe. SCN5A ΔKPQ ^{m/+} ES cells were obtained by transient expression of the Cre recombinase. Analytical restriction digest used for identification of recombinant ES cell clones was done by EcoRV.

Targeted SCN5A △KPQ^{m/+} ES clones were used to generate chimeric mice via morula aggregation, that were test bred with Swiss females for germline transmission. Viable germline offspring were born, and obtained at a normal Mendelian inheritance. Correct modification of the SCN5A gene was performed by sequencing isolated genomic DNA and mRNA. For sequencing genomic DNA, the following primers were used: forward primer 5'- GCA GTG GGA GGA CAA CCT CTA CAT G -3'and as reverse primer 5'-GTT CCA GCT GTT GGT GAA GTA ATA GTG G -3'. For sequencing rtPCR, the following primers were used: forward primer 5'- GCC TCA TCT TCT GGC TCA TCT TCA G-3' and as reverse primer 5'- GTT GTC AAA GTT GAC CTT CAC CTT GG-3'. SCN5A ΔKPQ^{m/+}mice were intercrossed by brother-sister mating to obtain homozygous SCN5A ΔKPQ/ ΔKPQ (^{m/m}) embryos. All homozygous embryos died at embryonic day 10 of development. All methods of ES culture, selection, and diploid aggregation have been described (Carmeliet et al. Nature 380, 439-442, 1996).

### Phenotypic analysis of SCN5A^{m/+} mice

### a) histological analysis

Since LQT3 is a dominant treat, we focused on heterozygous mice which are represented following normal Mendelian inheritance. These mice have a normal viability and no structural cardiac abnormalities were observed. Immunohistochemical analysis for Desmin, Laminin and Sercall revealed no differences between wild type and mutant mice. Tunel and Sirius red staining excluded increased apoptosis or fibrosis respectively. Myocardial wall thickness and dry-to-wet weight ratio measurements excluded dilatation or hypertrophy.

### b) electrocardiographic measurements in vivo

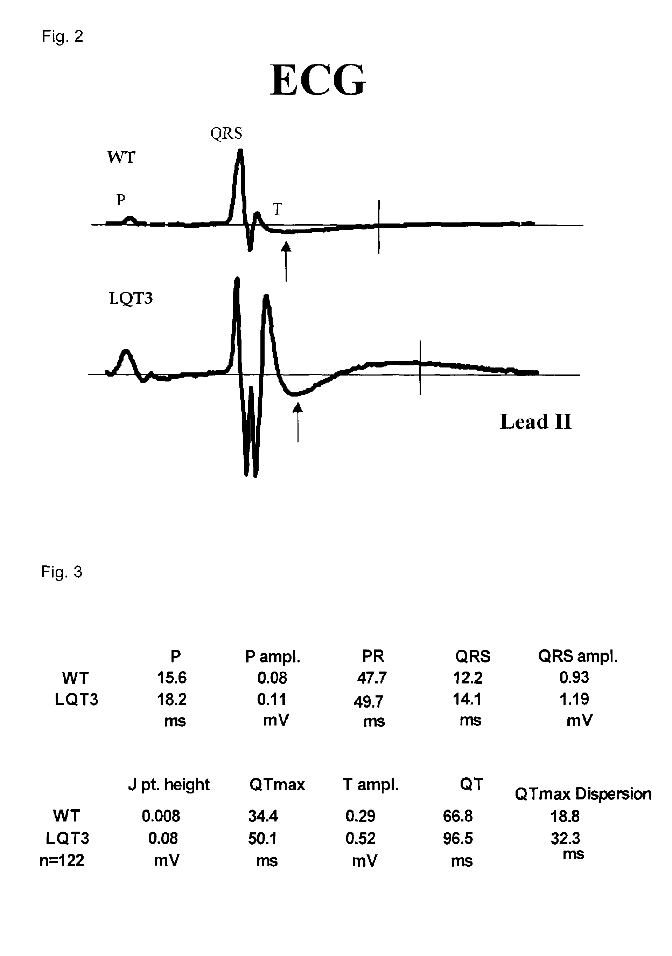

Eight-lead Electrocardiographic (ECG) analysis was performed. Animals were anaesthetised by intraperitoneal injection of sodium pentobarbital (100mg/kg). Eight lead ECGs were measured using 6 needle electrodes. Four electrodes were subcutaneously attached to the limbs, two electrodes were attached to the thorax at precordial position V2 en V6. All eight leads, (I, II, III, aVL, aVR, aVF, V2 and V6) were amplified with a Powerlab 16S amplifier and recorded digitally (4 kHz sampling / channel) using the Chart v3.6 program (AD Instruments). Signal Averaged Electrocardiogram (SAECG) was calculated using the Mouse SAECG extension v1.2 (AD Instruments) using the template matching algorithm (Fig. 2). This SAECG was used to calculate P, PR, QRS, QTmax, QT for every lead. Figure 3 summarizes the SAECG measurements. Dispersion of QTmax was measured as the difference between the longest and shortest QTmax interval. QTmax was semi-automatically calculated as the duration between the onset of the QRS complex and the maximum amplitude of the T wave. The end of the QT interval was defined as the duration between the onset of the QRS complex and the point after QTmax where the first derivative becomes zero. Mouse temperature was continuously monitored and recorded. For baseline measurements mice were heated to maintain a rectal temperature of 37°C. To induce slow heart rates, mice were cooled down to a rectal temperature of 28°C.
The following abnormalities were revealed at an average heart rate of LQT3: 349 (±72) BPM, WT 370 (±88) BPM:
- Prolonged QTmax and QT: all mice and all 8 leads averaged QTmax: LQT3 51,1 (±12,2) ms, WT 35,9 (±6,4) ms (p=5E-14, n=122) QT: LQT3 102,4 (±22,1) ms, WT 77,7 (±16,1) ms (p=1E-10, n=122)
- This prolongation becomes even more prominent at slow heart rates QTmax at 250 BPM: LQT3 58,8 (±17,5) ms, WT 38,9 (±4,8) ms (p=0,015, n=19) QT at 250 BPM: LQT3 129,3 (±18,9) ms, WT 86,3 (±13,6) ms (p=0,0001, n=19)
- Sharp Tall T-wave, mimicking morphology in patients: T amplitude in V2: LQT3 0,52 (±0,26) mV, WT 0,29 (±0,18) mV (p=2E-07, n=122)
- Increased dispersion, representing inhomogeneous repolarisation: QTmax dispersion over 8 leads: LQT3 32,3 (±19,5) ms, WT 18,8 (±13,6) ms (p=2E-05, n=122). QTmax and QT prolongation represent prolonged repolarisation.
Together with the increased dispersion this is a well known arrhytmogenic mechanism. The fact that these abnormalities are rate dependent, confirms the clinical finding that arrhythmias in LQT3 patients, happen more at slow heart rates.

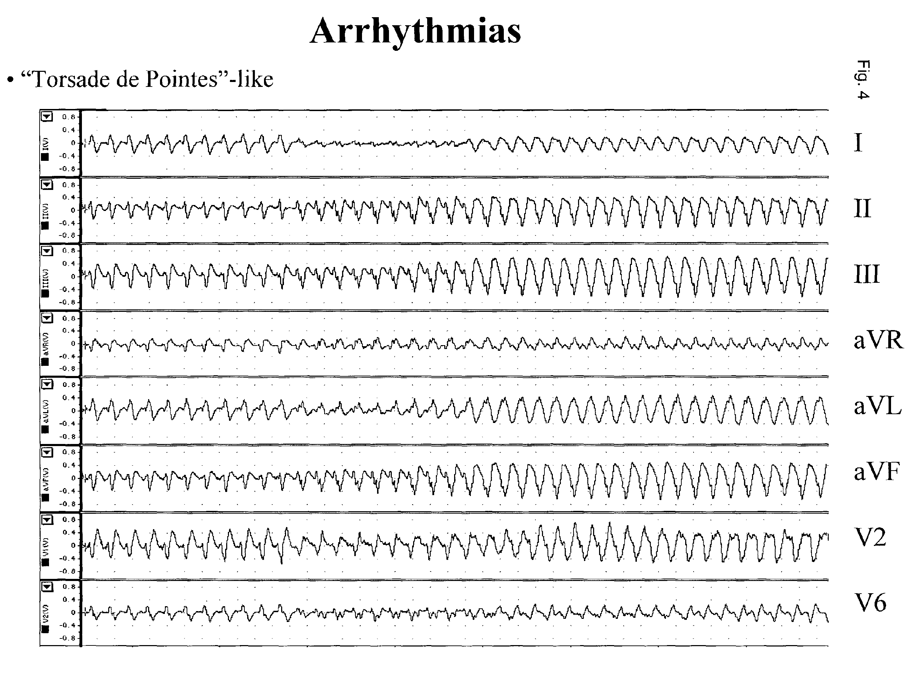

Arrhythmia detection on long recording periods revealed following abnormalities:
- "Torsade de Pointes" development in one LQT3 mouse (Fig. 4), in patients this polymorf ventricular tachycardia represents a life-threatening arrhythmogenic event (n=33) and is a hallmark for LQTS.
- Ventricular Extrasystoles were seen in approximately 10 of 33 LQT3 mice.
- AV block appeared in 4 of 33 LQT3 mice at very slow heart rate, but never in WT mice (n=33)
- Sudden axis changes or sudden changes in QRS morphology were found in almost all LQT3 mice, never in WT mice.
In summary these signs clearly represent a greater tendency to arrhythmia development in LQT3 mice.

### c) generation of homozygous mutant mice

Since all LQT3 mice were viable we were able to generate homozygous KPQ mice by intercrossing. Homozygous mice died during day 10 of embryologic development. At day 9.5 their hearts reveal weak and irregular contractions. Morphologic analysis revealed no major abnormalities.

### References

1. Wollnik, B. & Guicheney, P. Clinical and genetical aspects of the long QT syndrome. *Herz* **19**, 126-132 (1994).
2. Roden, D. M., Lazzara, R., Rosen, M., Schwartz, P. J., Towbin, J. & Vincent, G. M. Multiple mechanisms in the long-QT syndrome. Current knowledge, gaps, and future directions. The SADS Foundation Task Force on LQTS. *Circulation* **94,** 1996-2012 (1996).
3. Drugs causing prolongation of QT interval and torsade de pointes [news]. *Cmaj* **158**, 103-104, 107-108 (1998).
4. Wang, Q., Chen, Q. & Towbin, J. A. Genetics, molecular mechanisms and management of long QT syndrome. *Ann Med* **30,** 58-65 (1998).
5. Babij, P., Askew, G. R., Nieuwenhuijsen, B., Su, C. M., Bridal, T. R., Jow, B., Argentieri, T. M., Kulik, J., DeGennaro, L. J., Spinelli, W. & Colatsky, T. J. Inhibition of cardiac delayed rectifier K⁺ current by overexpression of the long-QT syndrome HERG G628S mutation in transgenic mice [In Process Citation]. *Circ Res* **83,** 668-678 (1998).
6. Barry, D. M., Xu, H., Schuessler, R. B. & Nerbonne, J. M. Functional knockout of the transient outward current, long-QT syndrome, and cardiac remodeling in mice expressing a dominant-negative Kv4 alpha subunit. *Circ Res* **83,** 560-567 (1998).
7. Drici, M. D., Arrighi, I., Chouabe, C., Mann, J. R., Lazdunski, M., Romey, G. & Barhanin, J. Involvement of IsK-associated K+ channel in heart rate control of repolarization in a murine engineered model of Jervell and Lange- Nielsen syndrome. *Circ Res* **83,** 95-102 (1998).
8. London, B., Jeron, A., Zhou, J., Buckett, P., Han, X., Mitchell, G. F. & Koren, G. Long QT and ventricular arrhythmias in transgenic mice expressing the N terminus and first transmembrane segment of a voltage-gated potassium channel. *Proc Natl Acad Sci U S A* **95,** 2926-2931 (1998).
9. Moss, A. J., Zareba, W., Benhorin, J., Locati, E. H., Hall, W. J., Robinson, J. L., Schwartz, P. J., Towbin, J. A., Vincent, G. M. & Lehmann, M. H. ECG T-wave patterns in genetically distinct forms of the hereditary long QT syndrome [see comments]. *Circulation* **92,** 2929-2934 (1995).
10. Dumaine, R., Wang, Q., Keating, M. T., Hartmann, H. A., Schwartz, P. J., Brown, A. M. & Kirsch, G. E. Multiple mechanisms of Na⁺ channel--linked long-QT syndrome. *Circ Res* **78,** 916-924 (1996).
11. Priori, S. G., Napolitano, C., Cantu, F., Brown, A. M. & Schwartz, P. J. Differential response to Na⁺ channel blockade, beta-adrenergic stimulation, and rapid pacing in a cellular model mimicking the SCN5A and HERG defects present in the long-QT syndrome. *Circ Res* **78**, 1009-1015 (1996).
12. Priori, S. G., Napolitano, C., Paganini, V., Cantu, F. & Schwartz, P. J. Molecular biology of the long QT syndrome: impact on management. *Pacing Clin Electrophysiol* **20,** 2052-2057 (1997).
13. Schwartz, P. J., Stramba-Badiale, M., Segantini, A., Austoni, P., Bosi, G., Giorgetti, R., Grancini, F., Marni, E. D., Perticone, F., Rosti, D. & Salice, P. Prolongation of the QT interval and the sudden infant death syndrome [see comments]. *N Engl J Med* **338,** 1709-1714 (1998).
14. Viskin, S. & Belhassen, B. Polymorphic ventricular tachyarrhythmias in the absence of organic heart disease: classification, differential diagnosis, and implications for therapy. *Prog Cardiovasc Dis* **41**, 17-34 (1998).
15. Deal, K. K., England, S. K. & Tamkun, M. M. Molecular physiology of cardiac potassium channels. *Physiol Rev* **76**, 49-67 (1996).
16. Dausse, E., Denjoy, I., Kahlem, P., Bennaceur, M., Faure, S., Weissenbach, J., Coumel, P., Schwartz, K. & Guicheney, P. Readjusting the localization of long QT syndrome gene on chromosome 11 p15. C *R Acad Sci III* **318,** 879-885 (1995).
17. Curran, M. E., Splawski, I., Timothy, K. W., Vincent, G. M., Green, E. D. & Keating, M. T. A molecular basis for cardiac arrhythmia: HERG mutations cause long QT syndrome. *Cell* **80**, 795-803 (1995).
18. Wang, Q., Shen, J., Splawski, I., Atkinson, D., Li, Z., Robinson, J. L., Moss, A. J., Towbin, J. A. & Keating, M. T. SCN5A mutations associated with an inherited cardiac arrhythmia, long QT syndrome. *Cell* **80**, 805-811 (1995).
19. Schott, J. J., Charpentier, F., Peltier, S., Foley, P., Drouin, E., Bouhour, J. B., Donnelly, P., Vergnaud, G., Bachner, L., Moisan, J. P. & et al. Mapping of a gene for long QT syndrome to chromosome 4q25-27. *Am J Hum Genet* **57**, 1114-1122 (1995).
20. Duggal, P., Vesely, M. R., Wattanasirichaigoon, D., Villafane, J., Kaushik, V. & Beggs, A. H. Mutation of the gene for IsK associated with both Jervell and Lange-Nielsen and Romano-Ward forms of Long-QT syndrome. *Circulation* **97**, 142-146 (1998).
21. Ackerman, M. J. The long QT syndrome. *PediatrRev* **19**, 232-238 (1998).
22. Wang, Q., Li, Z., Shen, J. & Keating, M. T. Genomic organization of the human SCN5A gene encoding the cardiac sodium channel. *Genomics* **34**, 9-16 (1996).
23. Schwartz, K. Impact of molecular genetics in cardiac diseases. *Herz* **19,** 69-74 (1994).
24. Vetter, D. E., Mann, J. R., Wangemann, P., Liu, J., McLaughlin, K. J., Lesage, F., Marcus, D. C., Lazdunski, M., Heinemann, S. F. & Barhanin, J. Inner ear defects induced by null mutation of the isk gene. *Neuron* **17**, 1251-1264 (1996).
25. Carmeliet, P., Stassen, J. M., Schoonjans, L., Ream, B., van den Oord, J. J., De Mol, M., Mulligan, R. C. & Collen, D. Plasminogen activator inhibitor-1 gene-deficient mice. II. Effects on hemostasis, thrombosis and thrombolysis. *J Clin Invest* **92**, 2756-2760 (1993).
26. Ploplis, V. A., Carmeliet, P., Vazirzadeh, S., Van Vlaenderen, I., Moons, L., Plow, E. F. & Collen, D. Effects of disruption of the plasminogen gene on thrombosis, growth, and health in mice. *Circulation* **92,** 2585-2593 (1995).
27. Carmeliet, P., Ferreira, V., Breier, G., Pollefeyt, S., Kieckens, L., Gertsenstein, M., Fahrig, M., Vandenhoeck, A., Kendraprasad, H., Eberhardt, C., Declercq, C., Pawling, J., Moons, L., Collen, D., Risau, W. & Nagy, A. Abnormal blood vessel development and lethality in embryos lacking a single vascular endothelial growth factor allele. *Nature* **380,** 435-439 (1996).
28. Carmeliet, P., Mackman, N., Moons, L., Luther, T., Gressens, P., Van Vlaenderen, I., Demunck, H., Kasper, M. , Breier, G. , Evrard, P., Müller, M. , Risau, W., Edgington, T. & Collen, D. Role of tissue factor in embryonic blood vessel development. *Nature* **383,** 73-75 (1996).
29. Dewerchin, M., Nuffelen, A. V., Wallays, G., Bouche, A., Moons, L., Carmeliet, P., Mulligan, R. C. & Collen, D. Generation and characterization of urokinase receptor-deficient mice. J *Clin Invest* **97**, 870-878 (1996).
30. Carmeliet, P. & Collen, D. Gene manipulation and transfer of the plasminogen system and coagulation system in mice. *Sem Thromb Hemost* **22,** 525-542 (1996).
31. Carmeliet, P., Moons, L., Lijnen, R., Baes, M., Crawley, J., Lemaître, V., Tipping, P., Drew, A., Eeckhout, Y., Shapiro, S., Lupu, F. & Collen, D. Urokinase-generated plasmin is a candidate activator of matrix metalloproteinases during atherosclerotic aneurysm formation. *Nature Genetics* **17**, 439-446 (1997).
32. Rosen, E., Chan, J.Y., Esohe, I., Clotman, F., Vlasuk, G., Albrecht, S., Lissens, A., Luther, T., Jalbert, L., Schoonjans, L., Moons, L., Collen, D., Castellino, F.J. & Carmeliet, P. Factor VII deficient mice develop normally but suffer fatal perinatal bleeding. *Nature* **390**, 290-294 (1997).
33. Baes, M., Gressens, P., Baumgart, E., Carmeliet, P., Casteels, M., Fransen, M., Evrard, P., Fahimi, D., Declercq, P. E., Collen, D., van Veldhoven, P. P. & Mannaerts, G. P. A mouse model for Zellweger syndrome. *Nat Genet* **17**, 49-57 (1997).
34. Carmeliet, P. & Collen, D. Genetic analysis of blood vessel formation. Role of endothelial versus smooth muscle cells. *Trends Cardiovasc Med* **7**, 271-281 (1997).
35. Carmeliet, P., Dor, Y., Herbert, J.M., Fukumura, D., Brusselmans, K., Dewerchin, M., Neeman, M., Bono, F., Abramovitch, R., Maxwell, P., Koch, C., Ratcliffe, P., Moons, L., Jain, R.K., Collen, D. & Keshet, E. Role of HIF-1alpha in hypoxia-mediated apoptosis, cell proliferation, and tumor angiogenesis. *Nature* **394,** 485-490 (1998).
36. Moons, L., Shi, V., Ploplis, V., Plow, E., Haber, E., Collen, D. & Carmeliet, P. Reduced transplant arteriosclerosis in plasminogen deficient mice. *J Clin Invest* **(in press)**(1998).
37. Carmeliet, P. & Collen, D. Vascular development and disorders: molecular analysis and pathogenetic insights. *Kidney Int* **53**, 1519-1549 (1998).
38. Bajou, K., Noel, A., Gerard, R. D., Masson, V., Brunner, N., Holst-Hansen, C., Skobe, M., Fusenig, N. E., Carmeliet, P., Collen, D. & Foidart, J. M. Absence of host plasminogen activator inhibitor 1 prevents cancer invasion and vascularization. *Nat Med* **4**, 923-928 (1998).
39. Carmeliet, P., Schoonjans, L., Kieckens, L., Ream, B., Degen, J., Bronson, R., De Vos, R., van den Oord, J. J., Collen, D. & Mulligan, R. C. Physiological consequences of loss of plasminogen activator gene function in mice. *Nature* **368,** 419-424 (1994).
40. Carmeliet, P., Moons, L., Ploplis, V., Plow, E. F. & Collen, D. Impaired arterial neointima formation in mice with disruption of the plasminogen gene. J *Clin Invest* **99,** 200-208 (1997).
41. Carmeliet, P., Moons, L. & Collen, D. Mouse models of angiogenesis, restenosis, atherosclerosis and hemostasis. *Cardiovasc Res* **39,** 8-33 (1998).
42. Carmeliet, E. & Zaman, M. Y. Comparative effects of lignocaine and lorcainide on conduction in the Langendorff-perfused guinea-pig heart. *Cardiovasc Res* **13,** 439-449 (1979).
43. Carmeliet, E. Chloride ions and the membrane potential of Purkinje fibres. *J Physiol* **156**, 375-388 (1961).
44. Tytgat, J., Vereecke, J. & Carmeliet, E. A combined study of sodium current and T-type calcium current in isolated cardiac cells. *Pflugers Arch* **417**, 142-148 (1990).
45. Carmeliet, E. Voltage- and time-dependent block of the delayed K+ current in cardiac myocytes by dofetilide. *J Pharmacol Exp Ther* **262***,* 809-817 (1992).
46. Carmeliet, E. Use-dependent block and use-dependent unblock of the delayed rectifier K+ current by almokalant in rabbit ventricular myocytes. *Circ Res* **73,** 857-868 (1993).
47. Gruber, R., Vereecke, J. & Carmeliet, E. Dual effect of the local anaesthetic penticainide on the Na⁺ current of guinea-pig ventricular myocytes. *J Physiol (Lond)* **435**, 65-81 (1991).
48. Carmeliet, E. Slow inactivation of the sodium current in rabbit cardiac Purkinje fibres. *Pflugers Arch* **408,** 18-26 (1987).
49. Carmeliet, E. Voltage-dependent block by tetrodotoxin of the sodium channel in rabbit cardiac Purkinje fibers. *Biophys J* **51**, 109-114 (1987).
50. Nilius, B., Vereecke, J. & Carmeliet, E. Properties of the bursting Na channel in the presence of DPI 201-106 in guinea-pig ventricular myocytes. *Pflugers Arch* **413,** 234-241 (1989).
51. Carmeliet, E. & Saikawa, T. Shortening of the action potential and reduction of pacemaker activity by lidocaine, quinidine, and procainamide in sheep cardiac purkinje fibers. An effect on Na or K currents? *Circ Res* **50,** 257-272 (1982).
52. Carmeliet, E. & Tytgat, J. Agonistic and antagonistic effect of R56865 on the Na⁺ channel in cardiac cells. *Eur J Pharmacol* **196**, 53-60 (1991).
53. Carmeliet, E., Nilius, B. & Vereecke, J. Properties of the block of single Na⁺ channels in guinea-pig ventricular myocytes by the local anaesthetic penticainide. J *Physiol (Lond)* **409,** 241-262 (1989).
54. Carmeliet, P. , Ng, Y., Nuyens, D., Theilmeier, G., Brusselmans, K, Cornelissen, I., Ehler, E., Kakkar, V., Stalmans, I., Mattot, V., Perriard, J., Flameng, J., Moons, L., Nagy, A., Lupu, F., Collen, D., D'Amore, P. & Shima, D. Impaired myocardial angiogenesis and ischemic cardiomyopathy in mice lacking the VEGF164 and VEGF188 isoforms. *Nat Gen* **Submitted.**
55. Wickman, K., Nemec, J., Gendler, S. J. & Clapham, D. E. Abnormal heart rate regulation in GIRK4 knockout mice. *Neuron* **20,** 103-114 (1998).
56. Carmeliet, E. & Mubagwa, K. Antiarrhytmic drugs and cardiac ion channel: mechanisms of action. *Prog Biophys Mol Biol* **70**, 1-72 (1998).
57. Undrovinas, A. I., Fleidervish, I. A. & Makielski, J. C. Inward sodium current at resting potentials in single cardiac myocytes induced by the ischemic metabolite lysophosphatidylcholine. *Circ Res* **71**, 1231-1241 (1992).
58. Wu, J. & Corr, P. B. Palmitoyl carnitine modifies sodium currents and induces transient inward current in ventricular myocytes. *Am J Physiol* **266,** H1034-1046 (1994).
59. Maltsev, V. A., Wobus, A. M., Rohwedel, J., Bader, M. & Hescheler, J. Cardiomyocytes differentiated in vitro from embryonic stem cells developmentally express cardiac-specific genes and ionic currents. *Circ Res* **75,** 233-244 (1994).

## Claims

1. A non-human transgenic animal comprising a specific deletion of KPQ-residues in the gene product encoded by the SCN5A gene.

2. A transgenic animal according to claim 1 wherein said deletion has been introduced into its genome via homologous recombination in an embryonic stem cell.

3. A transgenic animal according to claims 1 and 2, further **characterized in that** said animal is heterozygous for said deletion, and exhibits characteristics of human Long QT syndrome.

4. A transgenic animal model according to any of claims 1-3 wherein said animal is a mouse.

5. A cell line derived from the transgenic animal according to claims 1-4.

6. A cell line according to 5 wherein said cell line is a stem cell line and/or an embryonic stem cell line.

7. A method for screening a therapeutic molecule for use in the treatment of cardiac arrhythmias comprising:
- administering said molecule to one of the cell lines of claims 5 and 6
- monitoring a therapeutic effect of said molecule

8. A method for screening a therapeutic molecule for use in the treatment of cardiac arrhythmias comprising:
- administering a molecule to the transgenic animal according to claims 1-4
- monitoring a therapeutic effect of said molecule

9. A molecule obtainable by a screening method according to claims 7 and 8.

10. A molecule according to claim 9 for use in the preparation of a medicament for the treatment of cardiac arrhythmias.

11. A method for monitoring cardiac arrhythmogenic side effects of medicaments comprising:
- administering said medicaments to the transgenic animal according to claims 1-4 and/or cell lines according to claims 5 and 6 and
- monitoring a cardiac arrhythmogenic effect
